# EUROPEAN PATENT SPECIFICATION

(11) **EP 1 484 033 B1**
(45) Date of publication and mention of the grant of the patent: **23.02.2011**
(21) Application number: 04076699.0
(22) Date of filing: 18.03.1999
(51) Int. Cl.: A61F 2/02, A61K 9/50, B01J 13/02, B32B 5/16

(54) **Liquid polymeric compositions for controlled release of bioactive substances**
Flüssige Polymerlosung zur Kontrollierten freisetzung von bioaktiven substanzen
Compositions polymeres liquides pour la liberation controlee de substances bioactives

(30) Priority: 19.03.1998 US 79574 P; 21.07.1998 GB 9815801
(43) Date of publication of application: 08.12.2004
(62) Divisional of application: 99911462.2
(73) Proprietor: Merck Sharp & Dohme Corp., Rahway, NJ 07065 (US)
(72) Inventor: Chern, Rey T., Rahway, NJ 07065 (US); Zingerman, Joel R., Rahway, NJ 07065 (US)
(74) Representative: Buchan, Gavin MacNicol

(56) References cited:
- SINGH U V; SISHT K S; RAO S; DEVI P U; UDUPA N: "Reduced toxicity and enhanced antitumor efficacy of plumbagin using poly(lactic-co-glycolic) biodegradeable injectable implant" INDIAN JOURNAL OF PHARMACOLOGY, vol. 29, 1997, page 168-172, XP002197869
- SHAH N H ET AL: "A BIODEGRADABLE INJECTABLE IMPLANT FOR DELIVERING MICRO AND MACROMOLECULES USING POLY(LACTIC-CO-GLYCOLIC) ACID (PLGA) COPOLYMERS" JOURNAL OF CONTROLLED RELEASE, ELSEVIER SCIENCE PUBLISHERS B.V. AMSTERDAM, NL, vol. 27, no. 2, 1 November 1993 (1993-11-01), pages 139-147, XP000400402 ISSN: 0168-3659

## Description

### FIELD OF THE INVENTION

The present invention relates to liquid polymeric compositions for controlled release of a bioactive substance, i.e, a hydrophobic bioactive substance, such as a liquid polymeric composition which can form a film encapsulated liquid, e.g., *in situ* and/or which can achieve a long-term sustained release in a patient or host (e.g., animal or human) such as plasma profiles showing high efficacy (greater than 70%, such as at least 80%, preferably at least 90%, e.g., about 100% efficacy for greater than 12 months and/or plasma levels sustained for at least 50 or about 60 days or at least about two months or at least about eight weeks, e.g., at least 90 days or about three months or about 12 weeks or at least 120 days or about four months or about 16 weeks, or at least 150 days or about five months or about 20 weeks, or even longer, e.g., up to a year or more; for instance, from 1 to 12 months.

The present invention further relates to a liquid polymeric composition consisting essentially of (1) 1-10% w/v bioactive substance (i.e., hydrophobic bioactive substance which is eprinomectin); (2) 1-10% w/v of a biologically acceptable "polymer" (that is "copolymer", a polymer polymerized by at least two comonomers) (i.e, poly(lactide-glycolide) copolymer) wherein the weight ratio of the polymer to the bioactive substance can be 1:1 or less, e.g., 0.3:1 to 1:1; and (3) a mixture of hydrophilic and lipophilic solvents wherein the volume ratio of the hydrophilic and lipophilic solvents is from 80:20 to 5:45 for instance 80:20 to 10:90 or 5:95, hydrophilic and lipophilic solvents, e.g., 65:35 to 35:65, and/or wherein the the water immiscible or lipophilic solvent is present in an amount of at least 16.5% by weight (e.g., including about 16.;465% by weight), such as at least 16.5% to 45% by weight, for instance at least 16.5% to 30% by weight (e.g., at least 29% by weight) or at least greater than 40% by weight (for instance and at least about 42-45% by weight); e.g., such compositions wherein there is less than 10% of the polymer and 1 to 10% of the bioactive active substance or about less than 7% (e.g., 6.7%) or 5% or less polymer, with the bioactive substance content at less than or equal to about 10% or 5%.

The present invention yet further relates to a liquid polymeric composition consisting essentially of the foregoing, wherein the liquid polymeric composition is capable of forming a film encapsulated liquid, e.g., *in situ*, and/or having long-term sustained release, wherein the term "consisting essentially of" is used in the sense attributed to it in patent documents, and the term is exclusionary as to ingredients which may impede the capability of the composition to so form a film encapsulated liquid.

The present invention still further relates to methods for making and describes using such compositions. For example, a method of making such compositions comprising admixing the aforementioned ingredients; for instance, preferably dissolving both the polymer and the bioactive substance (as opposed to suspending, encapsulating, or having present as a solid, the bioactive substance, which, while not necessarily excluded by the invention, may be less preferable to dissolving). Or, a method for using such compositions comprising administering to a patient or host (animal, e.g., mammal such as domesticated animal, for instance companion animal or feedstock animal, or human) an inventive composition.

These and other areas to which the invention relates will be apparent from the following text. Various documents are cited in the following text, without any admission that any of these documents are prior art as to the invention.

### BACKGROUND OF THE INVENTION

Biodegradable polymers have been used in parenteral controlled release formulations of bioactive compounds. In one approach the polymer is fabricated into microspheres that may be injected via syringe, and the bioative compound is entrapped within the microspheres. This approach has not proved to be practical in part due to the difficulty in the manufacturing procedure for producing sterile and reproducible products, and the high cost of manufacturing. In another approach the biodegradable polymer and the bioactive material are dissolved in a biocompatible water-miscible solvent to provide a liquid composition. When the liquid composition is injected into the body, the solvent dissipates into the surrounding aqueous environment, and the polymer forms a solid depot from which the bioactive material is released.

European Patent Application 0537559 concerns polymeric compositions having a thermoplastic polymer, rate modifying agent, water soluble bioactive material and water-miscible organic solvent. Upon exposure to an aqueous environment (e.g. body fluids) the liquid composition is capable of forming a biodegradable microporous, solid polymer matrix for controlled release of water soluble or dispersible bioactive materials over about four weeks. The thermoplastic polymer may be, among many listed, polylactide, polyglycolide, polycaprolactone or copolymers thereof, and is used in high concentration (45 to 50%). The rate modifying agent may be, among many others listed, glycerol triacetate (triacetin); however, only ethyl heptanoate is exemplified; and the amount of the rate modifying agent is no more than 15%.

Indeed, with respect to the patent literature, reference is made to:

| **U.S. PATENT NO.** | **INVENTOR** |
|---|---|
| 4,150,108 | Graham |
| 4,329,332 | Couvreur et al. |
| 4,331,652 | Ludwig et al. |
| 4,333,919 | Kleber et al. |
| 4,389,330 | Tice et al. |
| 4,489,055 | Couvreur et al. |
| 4,526,938 | Churchill et al. |
| 4,530,840 | Tice et al. |
| 4,542,025 | Tice et al. |
| 4,563,489 | Urist |
| 4,675,189 | Kent et al. |
| 4,677,191 | Tanaka et al. |
| 4,683,288 | Tanaka et al. |
| 4,758.435 | Schaaf |
| 4,857,335 | Bohm |
| 4,931,287 | Bae et al. |
| 5,178,872 | Ohtsubo et al. |
| 5,252,701 | Jarrett et al. |
| 5,275,820 | Chang |
| 5,478,564 | Wantier et al. |
| 5,540,912 | Roorda et al. |
| 5,447,725 | Damani et al. |
| 5,599,852 | Scopelianos et al. |
| 5,607,686 | Totakura et al. |
| 5,609,886 | Wantier et al. |
| 5,631,015 | Bezwada et al. |
| 5,654,010 | Herbert et al. |
| 5,700,485 | Johnson et al. |
| 5,702,717 | Berde et al. |
| 5,711,968 | Tracy et al. |
| 5,733,566 | Lewis |
| 4,938,763 | Dunn et al. |
| 5,077,049 | Dunn et al. |
| 5,278,201 | Dunn et al. |
| 5,278,202 | Dunn et al. |
| 5,288,496 | Lewis |
| 5,324,519 | Dunn et al. |
| 5,324,520 | Dunn et al. |
| 5,340,849 | Dunn et al. |
| 5,368,859 | Dunn et al. |
| 5,401,507 | Lewis |
| 5,419,910 | Lewis |
| 5,427,796 | Lewis |
| 5,487,897 | Polson et al. |
| 5,599,552 | Dunn et al. |
| 5,632,727 | Tipton et al. |
| 5,643,595 | Lexis |
| 5,660,849 | Polson et al. |
| 5,686,092 | Lewis et al. |
| 5,702,716 | Dunn et al. |
| 5,707,647 | Dunn et al. |
| 5,717,030 | Dunn et al. |
| 5,725,491 | Tipton et al. |
| 5,733,950 | Dunn et al. |
| 5,736,152 | Dunn et al. |
| 5,744,153 | Yewey et al. |
| 5,759,563 | Yewey et al. |
| 5,780,044 | Yewey et al. |

These documents tend to provide compositions that form a solid, gel or coagulated mass; for instance, a significant amount of polymer is contemplated in these documents, akin to European Patent Application 0537559.

Mention is also made of: Shah et al (J. Controlled Release. 1993, 27:139-147), as relating to formulations for sustained release of bioactive compounds containing various concentrations of poly(lactic-coglycolic) acid copolymer (PLGA) dissolved in vehicles such as triacetin; Lambert and Peck (J. Controlled Release, 1995, 33:189-195), as a study of the release of protein from a 20% PLGA solution in N-methylpyrrolidone exposed to aqueous fluid; and Shivley et al (J. Controlled Release, 1995, 33:237-243), as a study of the solubility parameter of poly(lactide-co-glycolide) copolymer in a variety of solvents, and the in vivo release of naltrexone from two injectable implants (5% naltrexone in either 57% PLGA and 38% N-methylpyrrolidone or 35% PLGA and 60% N-methylpyrrolidone).

There is nonetheless a need for long term sustained-release compositions, as well as polymeric compositions which can form film coated or encapsulated liquids,

### OBJECTS AND SUMMARY OF THE INVENTION

In contrast to previous compositions, it has surprisingly been found that a polymeric composition containing a substantially greater amount of water immiscible or lipophilic solvent and substantially less polymer than contemplated by the literature results in a formulation which tends to stay as a film-coated (encapsulated) liquid rather than form a solid, gel or coagulated mass (including "pore-containing" solids, gels or masses as in the literature). It does not appear that the use or amount of the lipophilic solvent and the low amount of polymer used in the liquid polymeric formulations of the invention is contemplated by the prior art.

Accordingly, an object of the invention can be any or all of: to provide a liquid polymeric composition including a bioactive substance, for instance, such a composition that has long-term sustained release and/or forms a film-coated or encapsulated liquid, as well as to provide methods for making and/or using such a composition.

The present invention provides liquid polymeric compositions for controlled release of a bioactive substance, i.e, a hydrophobic bioactive substance, such as a liquid polymeric composition which can form a film encapsulated liquid, e.g., *in situ* and/or which can achieve a long-term sustained release in a patient or host (e.g., animal or human) such as plasma profiles showing high efficacy (greater than 70%, such as at least 80%, preferably at least 90%, e.g., about 100% efficacy for greater than 12 months and/or plasma levels sustained for at least 50 or about 60 days or at least two months or at least eight weeks, e.g., at least 90 days or about three months or about 12 weeks or at least 120 days or about four months or about 16 weeks, or at least 150 days or about five months or about 20 weeks, or even longer, e.g., up to a year or more; or from 1 to 12 months or longer.

The present invention further provides a liquid polymeric composition consisting essentially of :(1) 1-10% w/v of at least one bioactive substance (i.e, hydrophobic bioactive substance which is eprinomectin); (2) 1-10% w/v of at least one biologically acceptable "polymer" (that is "copolymer", a polymer polymerized by at least two comonomers) (i.e, poly(lactide-co-glycolide) copolymer), for instance, wherein the weight ratio of the polymer to the bioactive substance can be 1:1 or less, e.g., 0.5:1 to 1:1; and (3) a mixture of at least one hydrophilic solvent and at least one lipophilic solvent, e.g., at least one biologically or physiologically or medically or veterinarily acceptable hydrophilic solvent and at least one biologically or physiologically or medically or veterinarily acceptable lipophilic solvent wherein the volume ratio of the hydrophilic and lipophilic (or hydrophobic) solvents is from 80:20 to 5:95, for instance 80:20 to 10:90 or 5:95, hydrophilic and lipophilic solvents, e.g., 65:35 to 35:65, and/or wherein the the water immiscible or lipophilic solvent is present in an amount of at least 16.5% by weight (e.g., including 16.465% by weight), such as at least 16.5% to 45% by weight, for instance at least 16.5% to 30% by weight (e.g., at least 29% by weight), or at least 20% or 25% by weight to 30%, 35%, 40% or 45% by weight, or at least greater than 40% by weight (for instance and at least 42-45% by weight); e.g., such compositions wherein there is less than 10% of the polymer and 1 to 10% of the bioactive active substance or less than 7% (e.g., 6.7%) or 5% or less polymer, with the bioactive substance content at less than or equal to about 10% or 5%.

The present invention yet further provides a liquid polymeric composition consisting essentially of the foregoing, wherein the liquid polymeric composition is capable of forming a film encapsulated liquid, e.g., *in situ*, and/or having long-term sustained release, wherein the term "consisting essentially or is used in the sense attributed to it in patent documents, and the term is exclusionary as to ingredients which may impede the capability of the composition to so form a film encapsulated liquid. Thus, for instance, an agent which would tend to cause the composition, e.g., *in situ*, to have one or more contrary properties, e.g., an agent which would tend to cause the composition to solidify, such as a curing agent, or to form pores, may not be desired in certain embodiments.

The present invention still further provides methods for making and using such compositions. For example, a method of making such compositions comprising admixing the aforementioned ingredients; for instance, preferably dissolving both the polymer and the bioactive substance (as opposed to suspending, encapsulating, or having present as a solid, the bioactive substance, which, while not necessarily excluded by the invention, may be less preferable to dissolving). Or, a method for using such compositions comprising administering to a patient or host (animal, e.g., mammal such as domesticated animal, for instance companion animal or feedstock animal, or human) an inventive composition.

The invention additionally provides methods consisting essentially of at least one step for making or using such compositions; wherein the liquid polymeric composition is capable of forming a film encapsulated liquid, e.g., *in situ*, and/or having long-term sustained release, wherein the term "consisting essentially of" is used in the sense attributed to it in patent documents, and the term is exclusionary as to ingredients which may impede the capability of the composition to so form a film encapsulated liquid. Thus, for instance, a step which would tend to cause the composition, e.g., *in situ,* to have one or more contrary properties, e.g., adding an agent which would tend to cause the composition to solidify, such as a curing agent, or to form pores, may not be desired in certain embodiments.

Accordingly, the invention provides an *in situ* formed film coated or encapsulated liquid implant capable of functioning as a delivery system of drugs, medicaments, and other biologically-active agents to tissues adjacent to or distant from the implant site. The biologically-active agent is preferably incorporated into the film coated or encapsulated liquid, and subsequently released into surrounding tissue fluids and to the pertinent body tissue or organ. The composition may be administered to the implant site by any suitable method for applying a liquid, as for example, by means of a syringe, needle, cannula, catheter, pressure applicator, and the like.

The biologically active agents is
Thus, an object of the invention can be to provide delivery of at least one active ingredient, regardless of whether the ingredient is water insoluble or immiscible; but, the invention is especially applicable to hydrophobic biologically active substances.

The biologically acceptable polymer has one or more or all of the following characteristics: be bioerodible by cellular action, biodegradable by action of non-living body fluid components, soften when exposed to heat but return to the original state when cooled and are capable of substantially dissolving or dispersing in a water-miscible carrier or solvent to form a solution or dispersion. Upon contact with an aqueous fluid and the polymer are capable of assisting in the formation of the film coated or encapsulated liquid. The constitution of poly(lactide-co-glycolide) copolymer ("PLAGA") can be akin to its use in the Examples below or in documents cited herein.

The solvents can be any biologically or physiologically or medically or veterinarily hydrophobic and water miscible solvents such as those recognized in documents cited herein.

The hydrophilic solvent may be chosen from propylene glycol, PEG, polyglycols such as polyethylene glycol 200, polyethylene glycol 300 and polyethylene glycol 400, di(ethylene glycol)ethyl ether (Transcutol), isopropylidene glycerol(Solketal), dimethyl isosorbide (Arlasolve DMI), propylene carbonate, glycerol, glycofural, pyrrolidones such as N-methyl pyrrolidone and 2-pyrrolidone, isopropylidene glycerol, di(propyleneglycol) methyl ether, and mixtures thereof. Other solvents may also be useful as the hydrophilic solvent. For instance, the hydrophilic solvent can be a C₂ to C₈ alkanol (e.g., ethanol, propanol, butanol), acetone, alkyl esters such as methyl acetate, ethyl acetate, ethyl lactate, alkyl ketones such as methyl ethyl ketone, dialkylamides such as dimethylformamide, dimethyl sulfoxide, dimethyl sulfone, tetrahydrofuran, cyclic alkyl amides such as caprolactam, decylmethylsulfoxide, oleic acid, propylene carbonate, aromatic amides such as N,N-diethyl-m-toluamide, and 1-dodecylazacycloheptan-2-one. The hydrophilic solvent can be a mixture of solvents.

The lipophilic or non-water-miscible or hydrophobic solvent may be chosen from triethyl citrate, Miglyol 812, Miglyol 840, Crodamol GTCC, triacetin or benzyl benzoate; and additional lipophilic solvents may be used, e.g., hydrophobic rate modifying agents or plasticizers such as fatty acids, triglycerides, triesters of glycerol, oils such as castor oil, soybean oil or other vegetable oils or derivatives thereof such as epoxidized or hydrogenated vegetable oils such as epoxidized soybean oil or hydrogenated castor oil, sterols, higher alkanols (e.g., C₆ or higher), glycerin and the like. The lipophilic solvent can be a mixture of solvents.

Other solvents can include: glycol ethers such as propylene glycol monomethyl ether, dipropylene glycol monomethyl ether and diethylene glycol ethyl ether, di(ethylene glycol)ethyl ether acetate, di(propylene glycol)methyl ether (Dowanol DPM), di(propylene glycol)methyl ether acetate, glycerol formal, glycofurol, isopropyl myristate, N,N,-dimethyl acetamide, PEG 300, propylene glycol, and polar, aprotic solvents such as DMSO.

(See, e.g., the Example, wherein for instance 0.25 75/25 PLGA was dissolved in glycerol formal to provide a 2.5 ml solution; in a separate flask 75/25 PLGA was dissolved in triacetin to provide a 2.5 ml solution; the two solutions were mixed and added to a flask containing 0.50 g active ingredient which was dissolved into the mixed PLGA solutions; the amount of triacetin present in the formulation to be about 42% by weight; other formulations contain as little as 6.7% to and 5% PLGA content with the drug content at 10% or 5%.)

When implanted, i.e., upon section, the inventive liquid formulation forms what appears to be, from gross examination of the host or patient into which the formulation is implanted, "a semi-solid depot with a skin made of polymer The depot, though, without necessarily wishing to be bound by any one particular theory, is not necessarily solid or semi-solid (as that term may be usually understood); but rather, is a film coated or encapsulated liquid (the polymer assisting in the skin formation). Over time, depot loses its vehicle(s) (solvent(s) and degradation of the polymer occurs.

While there is diffusion through the film (typically whitish in color in preferred embodiments), it is believed that there are no pores in the depot; and it likely that the liquid polymeric formulation does not form *in situ*, a solid, or a coagulated mass or a gelatinous mass. These beliefs are based on the fact that the amount of polymer in the inventive formulation is substantially less than that used in the prior art; the amount of water immiscible or lipophilic solvent present in inventive formulations is substantially greater than any "rate modifying agent" or similar solvent used in the prior art (allowing the core of the depot to remains liquid); and, as the active ingredient diffuses through the film (a very, very thin film, usually whitish in preferred embodiments), the polymer biodegrades. The inventive formulation, is well-suited for delivering lipophilic (hydrophobic) active ingredients.

These and other embodiments are disclosed or are obvious from and encompassed by, the following Detailed Description.

### BRIEF DESCRIPTION OF FIGURES

The following Detailed Description, given by way of example, may be understood in conjunction with the accompanying Figures, incorporated herein by reference, in which:
Figure 1 depicts plasma levels of 6-amino-3-cyano-1-(2,6-dichloro-4-sulfurpentafluorophenyl)-4-(trifluoromethylthio)pyrazolo in dogs treated with the formulation of Reference Example 1;
Figure 2 depicts plasma levels of ivermectin in cattles treated with three of the ivermection formulations Reference Example 2; and,
Figure 3 depicts plasma levels of eprinomectin in swine treated with eprinomectin formulations of Example 1.

### DETAILED DESCRIPTION

The present invention provides liquid polymeric compositions for delivering bioactive substance(s).

The present invention provides liquid polymeric compositions for controlled release of a bioactive substance, i.e, a hydrophobic bioactive substance, such as a liquid polymeric composition which can form a film encapsulated liquid, e.g., *in situ* and/or which can achieve a long-term sustained release in a patient or host (e.g., animal or human) such as plasma profiles showing high efficacy (greater than 70%, such as at least 80%, preferably at least 90%, e.g., about 100% efficacy for greater than 12 months and/or plasma levels sustained for at least 50 or about 60 days or at least two months or at least eight weeks, e.g., at least 90 days or about three months or about 12 weeks or at least 120 days or about four months or about 16 weeks, or at least 150 days or about five months or about 20 weeks, or even longer, e.g., up to a year or more; or from 1 to 12 months or longer.

The present invention further provides a liquid polymeric composition consisting essentially of: (1) 1-10% w/v of a bioactive substance (i.e, hydrophobic bioactive substance which is eprinomectin); (2) 1-10% w/v of a biologically acceptable "polymer" (that is "copolymer", a polymer polymerized by at least two comonomers) (i.e poly(lactide-co-glycolide) copolymer) wherein the weight ratio of the polymer to the bioactive substance can be 1:1 or less, e.g., 0.5:1 to 1:1; and (3) a mixture of at least one hydrophilic solvent and at least one lipophilic solvent, e.g., at least one biologically or physiologically or medically or veterinarily acceptable hydrophilic solvent and at least one biologically or physiologically or medically or veterinarily acceptable lipophilic solvent wherein the volume ratio of the hydrophilic and lipophilic (or hydrophobic) solvents is from 80:20 to 5:95, for instance 80:20 to 10:90 , hydrophilic and lipophilic solvents, e.g., 65:35 to 35:65, and/or wherein the the water immiscible or lipophilic solvent is present in an amount of at least 16.5% by weight (e.g., including 16.465% by weight), such as at least 16.5% to 45% by weight, for instance at least 16.5% to 30% by weight (e.g., at least 29% by weight), or at least 20% or 25% by weight to 30%, 35%, 40% or 45% by weight, or at least greater than 40% by weight (for instance and at least 42-45% by weight); e.g., such compositions wherein there is less than 10% of the polymer and 1 to 10% of the bioactive active substance or about less than 7% (e.g., 6.7%) or 5% or less polymer, with the bioactive substance content at less than or equal to about 10% or 5%.

The present invention yet further provides a liquid polymeric composition consisting essentially of the foregoing, wherein the liquid polymeric composition is capable of forming a film encapsulated liquid, e.g., *in situ,* and/or having long-term sustained release, wherein the term "consisting essentially of" is used in the sense attributed to it in patent documents, and the term is exclusionary as to ingredients which may impede the capability of the composition to so form a film encapsulated liquid. Thus, for instance, an agent which would tend to cause the composition, e.g., *in situ*, to have one or more contrary properties, e.g., an agent which would tend to cause the composition to solidify, such as a curing agent, or to form pores, may not be desired in certain embodiments.

The present invention still further provides methods for making and using such compositions, as herein discussed.

The polymers and the solvents employed in the invention can be as herein discussed.

The in situ formed implants may also provide a delivery system for biologically-active agents to adjacent or distant body tissues and organs. Biologically-active agents which may be used alone or in combination in the present compositions and implants include medicaments, drugs, or any suitable biologically-, physiologically- or pharmacologically-active substance which is capable of providing local or systemic biological or physiological activity in an animal, including a human, and which is capable of being released from the depot into an adjacent or surrounding aqueous fluid.

The biologically-active agent may be miscible in the polymer and/or solvent to provide a homogenous mixture with the polymer, or insoluble in the polymer and/or solvent to form a suspension or dispersion with the polymer. It is highly preferred that the biologically-active agent be combined with the remaining components of the inventive composition almost immediately prior to administration of the composition to the implant site. It is also preferred that the bioactive agent not be water-miscible, e.g., at best only slightly soluble in water or or having low solubility in water or being able to dissolve into the lipophilic (hydrophobic) solvent. It is further preferred that the bioactive agent will not contain functional groups which will interfere the polymer. These conditions are readily determined by those of skill in the art simply by comparing the structure of the bioactive agent and the reacting moieties of the polymer.

The composition and in situ formed implant contain the biologically-active agent in an amount effective to provide a desired biological, physiological, pharmacological and/or therapeutic effect, optionally according to a desired release profile, and/or time duration of release. It is further preferred that the biologically-active agent is included in the polymer composition in an amount effective to provide an acceptable solution or dispersion viscosity.

The biologically-active agent may be included in the compositions in the form of, for example, an uncharged molecule, a molecular complex, a salt, an ether, an ester, an amide, or other form to provide the effective biological or physiological activity.

The amount of bioactive agent suitable for use in a formulation according to the invention can be determined by the skilled artisan without any undue experimentation from the knowledge in the art, and this disclosure, taking into consideration factors typically considered by those skilled in the medical, veterinary or pharmaceutical arts, such as the species involved, the age, weight, general health, and sex of the host or patient or animal or human, and the condition being treated and the LD₅₀ and other characteristics of the bioactive substance.

Thus, administration of the composition of the invention ultimately will be accomplished according to the wisdom and protocol of the patient's or host's or animal's or human's attending health care professional such as a physician or veterinarian, or if appropriate, a dentist. Choice of the particular composition will depend upon the malcondition or condition to be treated, which choice will be made by the attending health care professional. Application by syringe, or other means for applying a liquid to or into a tissue may be employed. The amounts and concentrations of composition administered to the patient, host, animal or human will generally be sufficient to accomplish the task intended. For administration of bioactive agent, the amounts and release rates will follow recommendations of the manufacturer of the bioactive agent. Generally, the concentration of bioactive agent in the liquid polymer formulation can be from 0.01 mg per g of mixture

In certain embodiments, the present invention, provides a liquid polymeric composition for controlled release of eprinomectin according to claim 1.

In a certain preferred embodiment, the eprinomectin, is present in a concentration of 5 to 10% w/v.

In another preferred embodiment, polymer, i.e, the poly(lactide-co-glycolide) copolymer, is present in a concentration of 1 to 5% w/v.

In another preferred embodiment, the weight ratio of the polymer, i.e, poly(lactide-co-glycolide) copolymer, to the bioactive substance, i.e, a hydrophobic bioactive substance, is 0.5:1 to 1:1.

In yet another preferred embodiment the volume ratio of the hydrophilic and lipophilic solvents is from 65:35 to 35:65.

In another aspect of the present invention there is disclosed a method for controlled release of a bioactive substance, i.e, a hydrophobic bioactive substance, which comprises injecting an animal with a liquid polymeric composition described herein.

In addition to the foregoing, as used herein the following terms are as defined below, unless otherwise specified:

"Hydrophobic: bioactive substance" means eprinomectin

"Poly(lactide-co-glycolide)" means a copolymer of lactic and glycolic acids having a lactide:glycolide ratio of from 75:25 to 65:35. The lactic acid can bed-or 1- or dl-. The copolymer may be a single copolymer of a mixture of copolymers within the above-defined parameters.

"Hydrophilic solvent" means water miscible solvents, preferably those when mixed with water in a ratio from 1:9 to 9:1 form a single-phase solution. Examples of hydrophilic solvents suitable for the present invention include, but not limited to glycerol formal, glycofural, N-methyl pyrrolidone, 2-pyrrolidone, isopropylidene glycerol, di(propylene glycol) methyl ether, and mixtures thereof.

"Lipophilic solvent" means water immiscible solvents, preferably with a solubility in water of less than 10% at room temperature. Example of lipophilic solvents suitable for the present invention include, but not limited to triacetin, benzyl benzoate, and mixtures thereof.

The liquid composition of the present invention is capable of providing prolonged drug release once it is injected, without the bursting drug release typical of existing liquid injectable formulations. Without being bound by theory, it is hypothesized that upon injection the liquid formulation of the present invention initially forms a depot with a skin made of the polymer surrounding a liquid core (which may appear "semi-solid"), while some of the hydrophilic solvent diffuses away from the depot carrying the dissolved bioactive compound with it. The initial drug release from the depot is mostly by permeation through the skin. The permeability of the skin and the rate of initial drug delivery are controlled by the proportions of the hydrophilic and lipophilic solvents in the liquid vehicle, at given polymer and drug concentrations. Over time, the depot loses its liquid vehicles and degradation of the polymer gradually becomes a significant drug-release mechanism. Proper adjustment of the liquid formulation composition thus allows overlap of the permeation-controlled and erosion-controlled drug delivery and results in a flattened and extended drug-release profile over a long period of time. And thus, the depot biodegrades, without wishing to necessarily be bound by any one particular theory, without necessarily forming a solid or other physical form associated with prior art compositions.

The presence of the lipophilic solvent in the liquid composition of the present invention reduces the initial delivery of bioactive compound, thus eliminating the bursting drug release typical of existing liquid injectable formulations where a large portion of hydrophilic vehicle is used. The presence of the hydrophilic solvent facilitates the formation of the polymer skin while preventing precipitation of the bioactive compound, thus allowing a much higher level of drug delivery than possible when only lipophilic vehicles are used. In the present invention, the prefered hydrophilic:lipophilic solvent ratio is between 80:20 to 20:80, most preferably between 65:35 to 35:65.

Other factors that may influence the performance of the present liquid formulation include: (1) the polymer, i.e, PLGA polymer concentration, (2) the relative proportion of the bioactive compound and the polymer, (3) the comonomer, e.g., lactide:glycolide raito in the polymer, and (4) the molecular weight of the polymer. Factors (3) and (4) were well known in the art (see documents cited herein). This invention, however, differs considerably from the existing art, especially in aspects (1) and (2).

The liquid formulation of the present invention contains no more than 10% polymer, e.g., PLGA polymer in order to maintain a relatively constant drug delivery rate at the same time ensuring a reasonably long drug delivery duration (greater than 3 months). The concentration of polymer, i.e, PLGA in the present formulation is therefore in sharp contrast to the known formulations where substantially larger proportion of polymer such as PLGA polymer is prescribed. The concentration of the bioactive substance in the liquid formulation may be from 1% to 10%. The proportion of polymer, i.e, PLGA polymer relative to the bioactive compound is less than or equal to 1:1; a ratio that is also substantially below those commonly prescribed. Within the range described herein, higher polymer concentrations reduce the drug delivery rate, and increasing the polymer:bioactive compound ratio also reduces the delivery rate.

The liquid composition may be prepared by dissolving all the solid ingredients in the vehicle under normal manufacturing conditions used for sterile injectable products. The present composition may contain additional inert substances commonly used in parenteral formulations including, but not limited to, antimicrobial agents, antioxidants, and the like.

The instant liquid compositions are administered to a warm-blooded animal such as human, cattle, sheep, pigs, dogs, horses, cats, and the like (e.g., mammals such as humans and companion and feedstock animals) by intramuscular or subcutaneous injection. The formulations will be prepared to contain from 1 to 10% of the bioactive compound. For instance, at a preferred dose volume of about 1 ml to treat a cattle of 50 kg body weight the formulation contains from 50 to 100 mg of avermectin compound per ml of solution or 5 to 10% w/v. However, depending upon the activity of the compound and the animal being treated, concentrations as low as 1% of bioactive compound are usable.

The following examples are provided to illustrate the invention and are not to be construed as limiting the invention in any manner.

### REFERENCE EXAMPLE 1

### Preparation of long-acting injectable formulation containing 6-amino-3-cyano-1-(2,6-dichloro-4-aulfurpentafluorophenyl)-4-(trifiuoromethylthio) pyrazole

Poly(DL-lactide/glycolide) 75/25 (PLGA, 0.25 g) was dissolved in sufficient glycerol formal to provide a 2.5 ml solution. In a separate flask poly(DL-lactidelglycolide) 75/25 (0.25 g) was dissolved in sufficient triacetin to provide a 2.5 ml solution. The two PLGA solutions were mixed well and added to a flask containing the active ingredient (0.50 g). The contents of the flask were mixed until the active ingredient dissolved, and the resulting solution was sterile filtered into a vial and sealed.

### REFERENCE EXAMPLE 2

### Preparation of long-acting injectable formulation containing ivermectin

The general procedure of reference Example 1 was followed to provide the following ivermectin formulations:

| No. | drug content % w/v | PLGA content % w/v | Solvent ratio TA/GF* | polymer type |
|---|---|---|---|---|
| 1 | 10 | 10 | 20/80 | 7525 |
| 2 | 10 | 10 | 35/65 | 7525 |
| 3 | 10 | 6.7 | 50/50 | 7525 |
| 4 | 10 | 5 | 50/50 | 7525 |
| 5 | 10 | 5 | 50/50 | 5050 |

| | | | | |
|---|---|---|---|---|
| * TA = triacetin; GF = glycerol formal | | | | |

For comparison purposes, i.e., to illustrate just how much more solvent is used in the present invention in comparison to prior art compositions: In preparation 1 of this example, the triacetin, the lipophilic solvent, is present at about 16.45% by weight. In preparation 2 of this example, the triacetin, the lipophilic solvent is present at about 29% by weight. In preparation 3 of this example, the triacetin, the lipophilic solvent is present at about 42% by weight. In preparation 4 of this example, the triacetin, the Lipophilic solvent, is present at about 43% by weight.

### EXAMPLE 1

### Preparation of long-acting injectable formulation containing eprinomectin

The general procedure of Reference Example 1 was followed to provide the following eprinomectin formulations:

| No. | drug content % w/v | PLGA content % w/v | Solvent ratio TA/GF | polymer type |
|---|---|---|---|---|
| 1 | 10 | 10 | 50/50 | 7525 |
| 2 | 5 | 5 | 50/50 | 6535 |

For comparison purposes, i.e., to illustrate just how much more solvent is used in the present invention in comparison to prior art compositions: In preparation 2 of this example, the triacetin, the lipophilic solvent, is present at about 45% weight percent. And, it is noted that the lipophilic solvent, in preparations according to this invention can be 100% of the volume of solvents present, as discussed in the foregoing general description.

### REFERENCE EXAMPLE 3

### Activity of long-acting injectable formulation containing 6-amino-3-cyano-1-(2,6-dichloro-4sulfurpentafluorophenyl)-4-(trifluoromethylthio) pyrazole against fleas in dogs

Three beagle dogs were treated with the formulation of Reference. Example 1 at a single subcutaneous dose of 10 mg/kg. Dogs were fasted for at least 6 hours before and 6 hours after the treatment. On day 1 (day 0 = day of drug administration) the animals were infested with approximately 100 fleas. Animals were combed and fleas counted and removed approximately 48 hours after infestation. Animals were infested on days 12 and 26, and combed and fleas counted and removed approximately 48 hours after infestation. Infestation/counting were repeated approximately monthly.

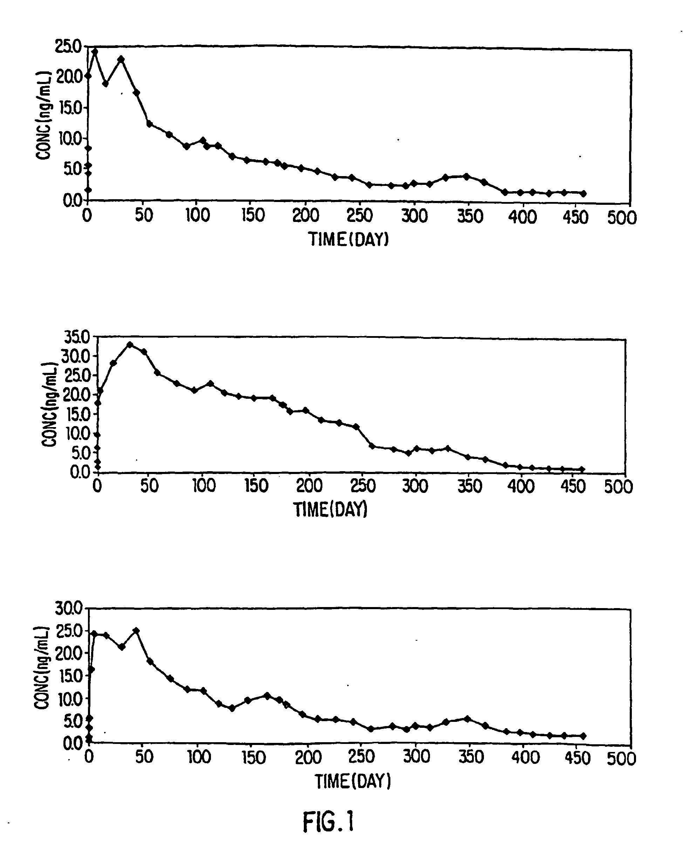

Blood samples were collected from animals on Day 0 at 1, 2, 3 and 6 hours after treatment, on Day 1 at 24 hours after treatment, and if emesis was observed. Blood samples were also collected when flea counts were determined. Animals were observed hourly for 6 hours post treatment for emesis. Close to 100% efficacy has been demonstrated for >12 months without any occurrence of emesis in the treated animals. The plasma level profiles for the individual dogs are shown as Figure 1.

### REFERENCE EXAMPLE 4

### Plasma level profiles of long-acting ivermectin formulations in cattle

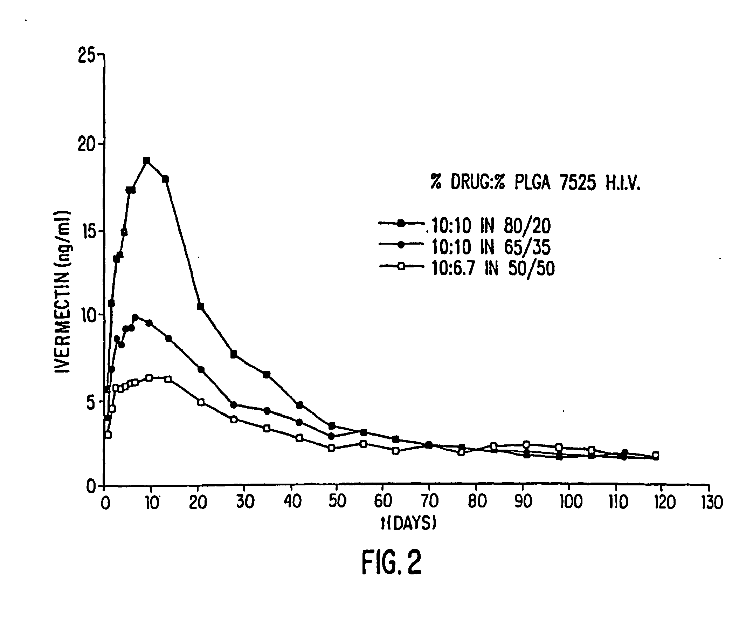

Plasma levels of ivermectin were determined in healthy cattles treated with ivermectin formulations 1, 2 and 3 of Reference Example 2. Each formulation was given to a group of five cattles (generally weighing 125 to 250 kg) as a single subcutaneous injection dosed at 1mg/kg. Ten ml heparinized blood samples were collected from each treated animal on Days 1-7 (daily), 10, 14, and weekly thereafter for 15 weeks. The plasma level profiles (average of the five animals in each group) are shown in Figure 2.

### EXAMPLE 2

### Plasma level profiles of long-acting eprinomectin formulation in swine

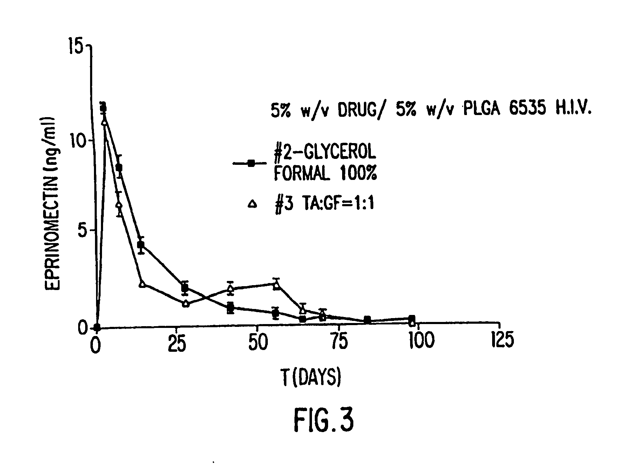

Plasma levels of eprinomectin were determined in swine treated with eprinomectin formulation 2 of Example 1. Three swine (inoculated with 2,000 infective ova of *Trichuris* suis on day -50, and orally with 15,000 infective larvae of Oesophagostomum sp. on day 0) were injected subcutaneously with formulation 2 of Example 1 at a dose of 1.5 mg/kg. Ten ml blood samples were collected from each animal on days 3, 7 and weekly thereafter. The plasma level profile is shown in Figure 3 (with alternate formulation of drug/PLGA in 100 glycerol formal).

## Claims

1. A liquid polymeric composition for controlled release of eprinomectin consisting essentially of:
(a) 1 to 10% w/v of eprinomectin;
(b) 1 to 10% w/v of poly(lactide-co-glycolide) copolymer;
wherein the weight ratio of the poly(lactide-co-glycolide) copolymer to the eprinomectin is 1:1 or less and the ratio of lactide:glycolide of the poly(lactide-co-glycolide) copolymer is from 75:25 to 65:35; and
(c) a mixture of hydrophilic and lipophilic solvents, wherein the volume ratio of the hydrophilic and lipophilic solvents is from 80:20 to 5:95.

2. The composition of claim 1 wherein die lipophilic solvent is triacetin.

3. The composition of claim 1 or 2 wherein the hydrophilic solvent is N-methyl pyrrolidone.

4. The composition of any preceding claim wherein (a) consists of about 5% w/v eprinomectin.

5. The composition of any preceding claim wherein the ratio of lactide:glycolide of the poly(lactide-co-glycolide) copolymer is about 75:25.

6. The composition of any preceding claim wherein (b) is 5% w/v poly(lactide-co-glycolide) copolymer.

7. Use of a composition as defined in any preceding claim for the manufacture of a medicament for treating a mammal.

8. The use of claim 7 wherein the mammal is bovine.

9. The use of claim 7 wherein the mammal is ovine.

10. The use of claim 7 wherein the mammal is canine.

11. A composition as defined in any one of claims 1 to 6 for use in a method of treatment as defined in claim 8, 9 or 10.

## Patentansprüche

1. Eine flüssige Polymerzusammensetzung zur kontrollierten Freisetzung von Eprinomectin, bestehend im Wesentlichen aus:
(a) 1 bis 10% Gew./Vol. Eprinomectin,
(b) 1 bis 10% Gew./Vol. eines Poly(lactid-co-glycolid)copolymers,
wobei das Gewichtsverhältnis des Poly(lactid-co-glycolid)copolymers zum Eprinomectin 1:1 1 oder weniger beträgt und das Lactid:Glycolid-Verhältnis des Poly(lactid-co-glycolid)copolymers 75:25 bis 65:35 beträgt, und
(c) einer Mischung aus hydrophilen und lipophilen Lösungsmitteln, wobei das Volumenverhältnis der hydrophilen und lipophilen Lösungsmittel von 80:20 bis 5:95 beträgt.

2. Die Zusammensetzung nach Anspruch 1, wobei das lipophile Lösungsmittel Triacetin ist.

3. Die Zusammensetzung nach Anspruch 1 oder 2, wobei das hydrophile Lösungsmittel N-Methylpyrrolidon ist.

4. Die Zusammensetzung nach einem vorhergehenden Anspruch, wobei (a) aus etwa 5% Gew./Vol. Eprinomectin besteht.

5. Die Zusammensetzung nach einem vorhergehenden Anspruch, wobei das Lactid:Glycolid-Verhältnis des Poly(lactid-co-glycolid)copolymers etwa 75:25 beträgt.

6. Die Zusammensetzung nach einem vorhergehenden Anspruch, wobei (b) 5% Gew./Vol. Poly(lactid-co-glycolid)copolymer ist.

7. Verwendung einer wie in einem vorhergehenden Anspruch definierten Zusammensetzung zur Herstellung eines Medikaments zur Behandlung eines Säugers.

8. Die Verwendung nach Anspruch 7, wobei der Säuger ein Rind ist.

9. Die Verwendung nach Anspruch 7, wobei der Säuger ein Schaf ist.

10. Die Verwendung nach Anspruch 7, wobei der Säuger ein Hund ist.

11. Eine wie in einem der Ansprüche 1 bis 6 definierte Zusammensetzung zur Verwendung bei einem wie in Anspruch 8, 9 oder 10 definierten Behandlungsverfahren.

## Revendications

1. Composition polymère liquide pour la libération contrôlée d'éprinomectine, consistant essentiellement en:
(a) 1 à 10% en p/v d'éprinomectine;
(b) 1 à 10% en p/v d'un copolymère de poly(lactide-co-glycolide);
où le rapport pondéral du copolymère de poly(lactide-co-glycolide) à l'éprinomectine est de 1:1 ou moins et le rapport en volume de lactide:glycolide du copolymère de poly(lactide-co-glycolide) est de 75:25 à 65:35; et
(c) un mélange de solvants hydrophile et lipophile où le rapport en volume des solvants hydrophile et lipophile est de 80:20 à 5:95.

2. Composition selon la revendication 1, dans laquelle le solvant lipophile est la triacétine.

3. Composition selon la revendication 1 ou 2, dans laquelle le solvant hydrophile est la N-méthylpyrrolidone.

4. Composition selon l'une quelconque des revendications précédentes, dans laquelle (a) consiste en environ 5% en p/v d'éprinomectine.

5. Composition selon l'une quelconque des revendications précédentes, dans laquelle le rapport de lactide:glycolide du copolymère de poly(lactide-co-glycolide) est d'environ 75:25.

6. Composition selon l'une quelconque des revendications précédentes, dans laquelle (b) est 5% en p/v de copolymère de poly(lactide-co-glycolide).

7. Utilisation d'une composition telle que définie dans l'une quelconque des revendications précédentes, dans la fabrication d'un médicament pour le traitement d'un mammifère.

8. Utilisation selon la revendication 7, dans laquelle le mammifère est de l'espèce bovine.

9. Utilisation selon la revendication 7, dans laquelle le mammifère est de l'espèce ovine.

10. Utilisation selon la revendication 7, dans laquelle le mammifère est de l'espèce canine.

11. Composition telle que définie dans l'une quelconque des revendications 1 à 6, à utiliser dans une méthode de traitement telle que définie dans la revendication 8, 9 ou 10.
